(19) Europäisches Patentamt
European Patent Office
Office européen des brevets

(11) EP 0 642 349 B1

(12) # EUROPÄISCHE PATENTSCHRIFT

(45) Veröffentlichungstag und Bekanntmachung des Hinweises auf die Patenterteilung:
**05.08.1998 Patentblatt 1998/32**

(21) Anmeldenummer: **93912706.4**

(22) Anmeldetag: **12.05.1993**

(51) Int. Cl.$^6$: **A61K 38/22**

(86) Internationale Anmeldenummer:
**PCT/EP93/01189**

(87) Internationale Veröffentlichungsnummer:
**WO 93/23070 (25.11.1993 Gazette 1993/28)**

(54) **ANWENDUNG VON URODILATIN BEI LUNGEN- UND BRONCHIALERKRANKUNGEN**
USE OF URODILATIN IN PULMONARY AND BRONCHIAL DISEASES
UTILISATION DE L'URODILATINE DANS LE TRAITEMENT DES MALADIES BRONCHO-PULMONAIRES

(84) Benannte Vertragsstaaten:
**AT BE CH DE DK ES FR GB GR IE IT LI LU MC NL PT SE**

(30) Priorität: **15.05.1992 DE 4216133**

(43) Veröffentlichungstag der Anmeldung:
**15.03.1995 Patentblatt 1995/11**

(73) Patentinhaber:
**HaemoPep Pharma GmbH**
**30625 Hannover (DE)**

(72) Erfinder:
- **FLÜGE, Thomas**
  **D-3000 Hannover 1 (DE)**
- **Forssmann, Wolf-Georg**
  **D-30175 Hannover (DE)**

(74) Vertreter:
**Meyers, Hans-Wilhelm, Dr.Dipl.-Chem. et al**
**Patentanwälte**
**von Kreisler-Selting-Werner**
**Postfach 10 22 41**
**50462 Köln (DE)**

(56) Entgegenhaltungen:
**WO-A-88/06596** **WO-A-92/21332**

- **BRITISH MEDICAL JOURNAL Bd. 299, Nr. 6707, 28. Oktober 1989, LONDON, GB Seiten 1081 - 1082 G. HULKS ET AL. 'BRONCHODILATOR EFFECT OF ATRIAL NATRIURETIC PEPTIDE IN ASTHMA.' in der Anmeldung erw hnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 642 349 B1

**Beschreibung**

Die vorliegende Erfindung betrifft die Verwendung des Peptidhormons Urodilatin zur Herstellung eines Arzneimittels zur Behandlung von Lungen- oder/und Bronchialerkrankungen.

Die Verwendung des Peptidhormons Urodilatin als blutdrucksenkendes Mittel ist auf dem pharmazeutischen Gebiet bekannt (DE 37 06 731.1; DE 37 17 329.4; PCT/EP88/00144).

Obstruktive Atemwegserkrankungen sind durch einen Spasmus der Bronchialmuskulatur, eine Schwellung der Bronchialschleimhaut und eine vermehrte Produktion von Bronchialsekret in unterschiedlicher Ausprägung gekennzeichnet. Sie umfassen insbesondere das Asthma bronchiale, die chronisch-obstruktiven Atemwegserkrankungen (COLD) und auch das Asthma cardinale. Zur Therapie von obstruktiven Atemwegserkrankungen ist die Verabreichung von $\beta_2$ Sypathomimetika (z.B. Fenoterol, Salbutamol, Terbutalin) bekannt. Die $\beta_2$-Sympathomimetika senken den Tonus der glatten Bronchialmuskulatur, sie hemmen darüber hinaus die Freisetzung von Mediatorsubstanzen aus den Mastzellen und steigern die mukoziliäre Klärfunktion. Eine langfristige und/oder hochdosierte Anwendung von $\beta_2$-Sympathomimetika kann jedoch zu einer Desensibilisierung von $\beta_2$-Adrenorezeptoren und damit zu einer starken Verringerung der therapeutischen Wirksamkeit führen.

Weiterhin ist die bronchodilatorische Wirksamkeit des atrialen natriuretischen Peptids (ANP) bei Asthma bekannt (Hulks et al., Br.Med.J. 299 (1989), 1081-1982).

Die Aufgabe der vorliegenden Erfindung bestand in der Bereitstellung eines neuen Therapeutikums für Lungen- oder/und Bronchialerkrankungen, insbesondere obstruktiven Atemwegserkrankungen, das anstelle von bekannten Therapeutika oder in Kombination mit diesen verwendet werden kann.

Die erfindungsgemäße Aufgabe wird durch die Bereitstellung einer pharmazeutischen Zusammensetzung, die Urodilatin als Wirkstoff und gegebenenfalls pharmazeutisch übliche Verdünnungs-, Träger-, Füll- oder Hilfsstoffe enthält, zur Behandlung von Lungen- oder/und Bronchialerkrankungen gelöst.

Die pharmazeutische Zusammensetzung eignet sich insbesondere zur Behandlung von obstruktiven Atemwegserkrankungen.

Die Zusammensetzung wird vorzugsweise parenteral, insbesondere intravenös (z.B. intravenöse Injektion (als Bolus) oder intravenöse Infusion) oder inhalatorisch, verabreicht, wobei die Dosierung vorzugsweise 5 ng bis 1000 µg Urodilatin pro kg Körpergewicht, besonders bevorzugt 10 ng bis 100 µg Urodilatin pro kg Körpergewicht beträgt.

In Tierversuchen konnte gezeigt werden, daß die parenterale Verabreichung von Urodilatin bei einer durch Inhalation von Acetylcholin hervorgerufenen Bronchokonstriktion zu einem deutlichen Schutz führt, der sich insbesondere durch eine verbesserte forcierte Exspiration zeigt.

Dabei wurde überraschenderweise festgestellt, daß die Wirkung von Urodilatin gegenüber äquimolaren Dosen von atrialem natriuretischem Peptid (ANP) um mehr als das zweifache höher war.

Weiterhin erhöht die intravenöse Applikation von Urodilatin die glomeruläre Filtrationsrate, die Wasser-, Natrium- und Chlorid-Ausscheidung ohne den bei gleicher Dosierung von ANP zu objektivierenden hypotensiven Effekt. Urodilatin ist daher vor allem bei kreislauflabilen Patienten mit einer geringeren Nebenwirkungsrate belastet. Die Relaxation der glatten Gefäßmuskulatur führt erst bei höheren Urodilatindosen zu einer Blutdrucksenkung.

Die geringeren hypotensiven Effekte von Urodilatin sollten bei einer möglichen Therapie einer Bronchokonstriktion im Vergleich mit ANP ebenfalls vorteilhaft sein. Besonders erscheint die Gefahr einer Wechselwirkung mit anderen hypotensiv wirkenden Medikamenten in der Therapie des Asthma bronchiale, z.B. Theophyllin-Präparaten, vermindert.

Die Erfindung wird weiterhin durch die folgenden Beispiele in Verbindung mit den Abbildungen 1 bis 12 verdeutlicht.

Es zeigen:

Abb. 1 Die forcierte exspiratorische Fluß-Volumen-Kurve am Ende der Acetylcholin-Provokation; gefüllter Kreis = Kontrolltiere, n = 7; leerer Kreis = Gruppe B: 40 ng/kg/min ≙ 11,4 pmol/kg/min Urodilatin, n = 6 (Mittelwerte ± SEM).

Abb. 2 Die forcierte exspiratorische Fluß-Volumen-Kurve am Ende der Acetylcholin-Provokation; gefüllter Kreis = Kontrolltiere, n = 7; leerer Kreis = Gruppe C: 80 ng/kg/min ≙ 22,8 pmol/kg/min Urodilatin, n = 6 (Mittelwerte ± SEM).

Abb. 3 Die forcierte exspiratorische Fluß-Volumen-Kurve am Ende der Acetylcholin-Provokation; gefüllter Kreis = Kontrolltiere, n = 7; leerer Kreis = Gruppe D: 70,3 ng/kg/min ≙ 22,8 pmol/kg/min ANP, n = 6 (Mittelwerte ± SEM).

Abb. 4 Die Kurve der exspiratorischen Ein-Sekunden-Kapazität ($FEV_{1,0}$) bei Behandlung einer Patientengruppe durch Infusion mit unterschiedlichen Konzentrationen von Urodilatin.

Abb. 5 Die Kurve der Vitalkapazität ($VC_{max}$) bei Behandlung einer Patientengruppe mit Urodilatin.

Abb. 6 Die Kurve des maximal-exspiratorischen Spitzenflusses (PEF) bei Behandlung einer Patientengruppe mit Urodilatin.

Abb. 7-9 Kurven für den Fluß bei 75, 50 bzw. 25 % der Vitalkapazität ($MEF_{75}$, $MEF_{50}$, $MEF_{25}$) bei Behandlung einer Patientengruppe mit Urodilatin.

Abb. 10 Die Kurve für den systolischen Blutdruck (BP) in mm Hg bei Behandlung einer Patientengruppe mit Urodilatin.

Abb. 11 Die Kurve für den diastolischen Blutdruck (BP) in mm Hg bei Behandlung einer Patientengruppe mit Urodilatin.

Abb. 12 Die Kurve für die Herzfrequenz (HR) in Schlägen/min (beats/min) bei Behandlung einer Patientengruppe mit Urodilatin.

Beispiel 1

1. Methoden

Als Versuchstiere dienten insgesamt 33 weibliche, 12 Wochen alte Wistar-Ratten mit einem Körpergewicht von 300 g. Das Design der Studie entsprach einem einfach blinden Vorgehen. An allen Versuchstagen wurden jeweils Tiere der Kontrollgruppe und der Verumgruppe gemessen.

Der Einsatz eines Versuchstierbodyplethysmographen erlaubte an der narkotisierten, intubierten und spontanatmenden Ratte fortlaufend die Messung von Atemzugvolumen (VT), Atemfrequenz (f), Atem-Minuten-Volumen (MV), Lungenresistance (RL), dynamischer Compliance (Cdyn), Atemfluß (F) und Transpulmonaldruck (PTP). Die Kreislaufüberwachung erfolgte durch EKG-Registrierung mit Auswertung der Herzfrequenz (HR).

Zur Applikation von Placebo (NaCl-Lösung) oder Verum (Urodilatin oder ANP in NaCl-Lösung) wurde den Versuchstieren ein intravenöser Zugang (Butterfly) in die Schwanzvene gelegt. Nach Erreichen von steady state-Bedingungen erfolgte zunächst die Messung der Ausgangswerte der Spontanatmung. Im Anschluß daran wurde randomisiert die Infusion von Placebo, Urodilatin oder ANP begonnen. Eine Auswertung der kontinuierlich bestimmten Spontanparameter wurde jeweils 5 Minuten nach Infusionsbeginn von einer inhalativen Acetylcholin (ACh)-Provokation durchgeführt (Basiswerte vor ACh-Provokation).

Mit einem Injektomat-Gerät wurde den Versuchstieren kontinuierlich über insgesamt 10 Minuten 0,5 ml Lösung infundiert. Die Kontrolltiere der Gruppe A erhielten 0,5 ml NaCl-Lösung, die Ratten der Gruppe B in NaCl-Lösung 40 ng/kg/min = 11.409 pmol/kg/min Urodilatin entsprechend 120 ng pro Tier, Gruppe C 80 ng/kg/min = 22.819 pmol/kg/min Urodilatin entsprechend 240 ng pro Tier und der Gruppe D 70.305 ng/kg/min = 22.819 pmol/kg/min ANP entsprechend 210.915 ng pro Tier. In den Gruppen C und D wurden demnach äquimolare Dosen von Urodilatin bzw. ANP appliziert. Vorversuche zeigten bei Infusion von 400 ng/kg/min Urodilatin in einem Volumen von 1 ml keine Veränderungen der Herzfrequenz der untersuchten Tiere.

5 Minuten nach Beginn der Infusion wurde jede Ratte durch Inhalation einer definierten Menge eines ACh-Aerosols provoziert. Die Auswertung der Spontanatmungsparameter erfolgte bei allen Tieren nach einem Gesamtinhalationsvolumen von 600 ml (Werte am Ende der ACh-Provokation).

Zum Abschluß der Versuche wurde bei jedem Tier in Hyperventilations-induzierter Apnoe eine forcierte Exspiration durchgeführt. Es erfolgte dabei die Registrierung der Fluß-Volumen (Flow-Volume)- und der Fluß-Zeit (Spirographie)-Relation. Die Flow-Volume-Kurve erlaubt die Auswertung von maximal-exspiratorischem Spitzenfluß (Peak Expiratory Flow = PEF), maximalem mitt-exspiratorischer Fluß (Maximal Mid-Expiratory Flow = MMEF) und dem Fluß bei 75, 50, 25 und 10 % der forcierten Vitalkapazität (z.B. FEF 75). Die Spirographie liefert bei der Ratte vor allem die forcierte Vitalkapazität (FVC) und das exspirierte Volumen nach 0,05, 0,10, 0,20 und 0,40 Sekunden als Absolutwert in ml (z.B. FEV 0,05) oder in Prozent der FVC (z.B. FEV 0,05 %).

2. Ergebnisse

2.1 Spontanatmungsparameter:

2.1.1 Vergleich der Ausgangswerte der Gruppen A-D:

Die Messung von F, PTP, VT, Cdyn, RL, f und MV unter Spontanatmung ergab bei den Ausgangswerten keine signifikanten Unterschiede zwischen den Gruppen A-D.

2.1.2 Infusion von NaCl, Urodilatin oder ANP:

Im Vergleich mit den Ausgangswerten kam es 5 Minuten nach Beginn der Infusion in der Gruppe B (40 ng/kg/min Urodilatin) zu einem leichten signifikanten Anstieg von PTP und RL und einem geringen signifikanten Abfall von Cdyn. In der Gruppe C (80 ng/kg/min Urodilatin) war gegenüber den Ausgangswerten ein leichter signifikanter Abfall der Atemfrequenz zu verzeichnen. Der Vergleich aller Gruppen untereinander (Anova) und einzeln gegenüber der Kontrollgruppe A (t-Test) ergab insgesamt für die Basiswerte vor der ACh-Provokation und für deren auf die Ausgangswerte bezogenen prozentualen Veränderung nach 5 Minuten Infusion (Delta %) keine signifikanten Unterschiede zwischen den Gruppen A-D.

2.1.3 ACh-Provokation unter Infusion von NaCl, Urodilatin oder ANP:

Die ACh-Provokation führte in allen Gruppen gegenüber den Basiswerten vor der Inhalation zu einem signifikanten Anstieg von PTP, RL, f und MV und einem signifikanten Abfall von VT und Cdyn. Außer in der Gruppe D kam es in allen andere Gruppen zu einem signifikanten Abfall von F. Der Vergleich aller Meßwerte und ihrer absoluten bzw. relativen Veränderungen gegenüber den Basiswerten vor ACh-Provokation zeigte nach einer Gesamtinhalation von 600 ml ACh zwischen den Gruppen (Anova) und im Vergleich der Gruppen B-D einzeln gegenüber der Kontrollgruppe A (t-Test) insgesamt keine signifikanten Veränderungen.

2.2 Forcierte Exspiration:

Die Meßparameter der forcierten Exspiration wurden nur am Ende der ACh-Provokation erhoben, so daß statistisch nur ein Vergleich zwischen den Gruppen A-D möglich ist.

2.2.1 Spirographie:

Bei den auf die FVC bezogenen Werte des exspirierten Volumens nach 0,05, 0,1, 0,2 und 0,4 Sekunden (FEV 0,5 % usw.) zeigte die multivariate Varianzanalyse (Anova) im Gruppenvergleich und der gepaarte t-Test der Gruppen B-D einzeln gegenüber der Kontrollgruppe A nur für FEV 0,1 % in der Gruppe B (40 ng/kg/min Urodilatin) eine signifikante Verbesserung. Die übrigen FEV- und FEV%-Werte in den Gruppen A-D waren nicht signifikant verschieden.

2.2.3 Fluß-Volumen-Kurven:

Der Vergleich der Gruppen A-D untereinander und einzeln gegenüber der Kontrollgruppe A zeigte für die Gruppen B (40 ng/kg/min Urodilatin) und C (80 ng/kg/min Urodilatin) eine deutliche Verbesserung von PEF, MMEF, FEF 75, FEF 50 und FEF 25. Die zur Gruppe C (80 ng/kg/min Urodilatin) äquimolare Dosis von ANP in der Gruppe D (70.305 ng/kg/min) bewirkte nur eine signifikante Zunahme von PEF und FEF 75 (Abbildungen 1 bis 3).

Die Abbildungen 1 bis 3 zeigen die forcierte exspiratorische Fluß-Volumen-Kurve am Ende der Acetylcholin-Provokation bei den Gruppen B (40 ng/kg/min Urodilatin), C (80 ng/kg/min Urodilatin) und D (70,3 ng/kg/min ANP). Aus den Abbildungen ist ersichtlich, daß Urodilatin (Gruppen B und C) stärker und länger wirksam ist als atriales natriuretisches Peptid (Gruppe D). Aus einem Vergleich der Abbildungen 1 und 3 geht hervor, daß Urodilatin bereits bei 40 ng/kg/min deutlich wirksamer als ANP in einer molar 2-fach höheren Dosis ist.

3.3 Herzfrequenz:

Die Unterschiede der Herzfrequenz vor Infusionsbeginn (Ausgangswerte), nach 5 Minuten Infusion (Basiswerte vor ACh-Provokation) und am Ende der ACh-Provokation waren zwischen den Gruppen A-D nicht signifikant.

3. Diskussion:

3.1 Spontanatmungsparameter:

Die leichten Veränderungen von PTP, RL und Cdyn gegenüber den Ausgangswerten unter der Infusion von 40 ng/kg/min Urodilatin (Gruppe B) erreichten im statistischen Vergleich der Gruppen untereinander keine Signifikanz und können vermutlich als Folgen der Volumenbelastung durch die Infusion mit konsekutiver Versteifung der Lunge und Zunahme des Atemwegswiderstandes durch Bronchialschleimhautschwellung bei besonders empfindlichen Tieren angesehen werden.

Die ACh-Provokation führte in allen Gruppen zu einer deutlichen Bronchokonstriktion mit Abnahme der Lungendehnbarkeit, ohne daß ein signifikanter Effekt der Urodilatin- oder ANP-Infusion auf die Spontanatmungsparameter objektiviert werden konnte.

3.2 Forcierte Exspiration:

Spirographie und Flow-Volume-Kurve sind als sensible Meßverfahren zur Bestimmung von Funktionsänderungen der großen zentralen und kleinen peripheren Atemwege anzusehen. FEV, FEV% und PEF reflektieren besser noch als die unter Spontanatmung bestimmte Resistance den Funktionszustand der zentralen Atemwege, während die Flow-Messungen bei 75, 50, 25 und 10 % der forcierten Vitalkapazität (FVC) zu kleineren Lungenvolumina hin zunehmend die Weite der peripheren Bronchien widerspiegeln. Die deutlichen protektiven Effekte der Infusion von 40 und 80 ng/kg/min Urodilatin auf die im Vergleich mit der Kontrollgruppe A unter Placebo-Infusion zu objektivierenden ACh-induzierten Veränderungen der Parameter der forcierten Exspiration können daher trotz des fehlenden Ansprechens der Spontanatmungsmeßwerte als signifikante Medikamentenwirkung angesehen werden. Die Effekte der zu 80 ng/kg/min Urodilatin äquimolaren Dosis von 70.305 ng/kg/min ANP waren im Vergleich weniger deutlich ausgeprägt. Bereits 40 ng/kg/min Urodilatin wirkten deutlicher protektiv als ANP in der molar 2-fach höheren Dosierung. Die vorwiegend peripher zu lokalisierende Wirkung von Urodilatin kann durch die Art der Applikation als intravenöse Infusion bedingt sein.

3.3 Herzfrequenz:

Ein wesentlicher Anstieg der Herzfrequenz als Folge einer Vasodilatation unter der Urodilatin- oder ANP-Infusion mit reflektorischer Sympathikotonuserhöhung konnte bei den applizierten Dosen nicht nachgewiesen werden. Die Urodilatin-bzw. ANP-induzierte Protektion gegenüber der ACh-Provokation ist somit als Pharmakonwirkung zu interpretieren.

Beispiel 2

1. Methoden:

18 Patienten der Asthma bronchiale-Ambulanz der Medizinischen Hochschule Hannover, 2 Frauen und 16 Männer, im Alter zwischen 20 und 61 Jahren (33,7 ± 14,8 (Mittelwert ± STD)) wurden untersucht. Sie wiesen alle ein gemischt-förmiges Asthma bronchiale auf, 4 Patienten litten zusätzlich an einem labilen Hypertonus. Die Anamnese ergab keine Hinweise auf eine Exazerbation der Erkrankung in den letzten 8 Wochen. Ein Nikotinabusus war bei keinem der Patienten bekannt. Als weitere Einschlußkriterien wurden eine exspiratorische Einsekunden-Kapazität ($FEV_{1,0}$) von 40 bis 70 % der maximalen Vitalkapazität ($VC_{max}$) vor Applikation eines Bronchospasmolytikums, einen Effekt eines β2-Sympathomimetikums von ≥ 15 % auf die $FEV_{1,0}$, keine Änderung der antiobstruktiven Dauermedikation und keine Einnahme von oralen Corticoiden in den 4 Wochen vor der Studie definiert. Innerhalb von 8 Stunden vor Beginn der Untersuchungen durfte keine Anwendung eines inhalativen β2-Sympathomimetikums mehr erfolgen.

Am Studientag wurden alle Messungen in der Zeit zwischen 9:00 und 13:00 Uhr durchgeführt, um Lungenfunktionsveränderungen durch circadiane Schwankungen auszuschalten. Zunächst erfolgte die Dokumentation der Erfüllung der Einschlußkriterien durch eine Basis-Lungenfunktionsuntersuchung (base). Die Bestimmung der Volumen-Zeit-Relation ergab die Meßwerte $FEV_{1,0}$ und $VC_{max}$, die Fluß-Volumen-Beziehung, den exspiratorischen Spitzenfluß (PEF = peak expiratory flow) und den Fluß bei 75, 50 und 25 % der Vitalkapazität ($MEF_{75}$, $MEF_{50}$, $MEF_{25}$ = maximal expiratory flow). Die körperliche Untersuchung schloß die Erhebung der Kreislaufparameter Blutdruck und Herzfrequenz ein. Um möglichen Flüssigkeitsverlusten durch die diuretische Wirkung von Urodilatin vorzubeugen, wurde allen Patienten über einen intravenösen Katheter am Unterarm über 30 Minuten 500 ml 0,9 %ige Kochsalzlösung infundiert.

Nach erneuter Bestimmung von Lungenfunktionsparametern, Blutdruck und Herzfrequenz (pre) erfolgte die Infusion von 20, 40 oder 60 ng Urodilatin pro kg Körpergewicht pro Minute (ng/kg/min) entsprechend 5,71, 11,41 oder 17,11 pmol/kg/min über 40 Minuten. Während dieser Zeit und über 30 Minuten nach Beendigung der Infusion wurden im Abstand von 10 Minuten die Messungen von Lungenfunktion, Blutdruck und Herzfrequenz wiederholt. Zur Dokumentation der maximal erreichbaren Bronchodilatation inhalierten die Patienten zum Schluß 1,25 mg Salbutamol (Sultanol®-Fertiginhalat). Die Lungenfunktions-und Kreislaufparameter wurden dann erneut zum Zeitpunkt der subjektiv maximalen Wirkung bestimmt (Salb).

2. Ergebnisse:

Die Resultate werden durch die Abbildungen 4 bis 12 dokumentiert. In diesen Abbildungen bedeuten: * = $p < 0,5$ △ zu pre-Wert; + = $p < 0,05$ zu 20 ng △ zu pre-Wert; (in Abb. 12: $p < 0,05$ △ zwischen Dosierungsgruppen); ‡ = $p < 0,05$ zu 40 ng △ zu pre-Wert; # = △ Uro-Max. zu Salb n.s..

Die Lungenfunktionsparameter und die Blutdruckwerte zeigten für die Messung vor Beginn der Infusion (pre) keine signifikanten Unterschiede zwischen den drei Dosisgruppen.

Die Meßparameter der Volumen-Zeit-Relation sind in den Abb. 4 und 5 dargestellt. Nach einer Infusionsdauer von 10 Minuten kam es unter 40 und 60 ng/kg/min Urodilatin bereits zu einem gegenüber den pre-Werten und den Meßdaten der Gruppe 20 ng/kg/min signifikanten Anstieg des $FEV_{1,0}$. 20 ng/kg/min Urodilatin zeigte zu keinem Zeitpunkt während oder nach der Infusion einen signifikanten Effekt. Das Maximum der Wirkung trat unter 60 ng/kg/min Urodilatin nach 20 Minuten auf und war nicht mehr signifikant verschieden von den Meßwerten nach Salbutamol-Inhalation (Salb). Die Patientengruppe mit der Dosierung 40 ng/kg/min zeigte die maximalen $FEV_{1,0}$-Werte nach 30 Minuten Infusion. Nach Beendigung der Infusion kam es zu einem verstärkten Abfall der exspiratorischen Einsekunden-Kapazität, so daß nur noch die Ergebnisse der Gruppe 40 ng/kg/min nach 30 Minuten signifikant von den Ausgangswerten verschieden waren. 1,25 mg Salbutamol führte in allen drei Gruppen zu einem signifikanten Anstieg des $FEV_{1,0}$.

Als Ausdruck der bronchospasmolytischen Wirkung von Urodilatin war während der Infusion von 40 und 60 ng/kg/min und bis zu 10 Minuten danach ein gegenüber den pre-Werten signifikanter Anstieg des $VC_{max}$ zu objektivieren. Die Maxima zeigten keine signifikanten Unterschiede zu den Ergebnissen nach Salbutamol-Inhalation.

Die Abb. 6 bis 9 dokumentieren den Verlauf der Meßparameter der Fluß-Volumen-Relation. Der PEF, $MEF_{75}$, $MEF_{50}$ und $MEF_{25}$ stiegen während der Infusion von 40 und 60 ng/kg/min Urodilatin an. Signifikante Unterschiede wurden im Vergleich zu den Ausgangswerten und gegenüber der Gruppe mit 20 ng/kg/min Urodilatin erreicht. Die Maxima unter Infusion waren mit Ausnahme des $MEF_{50}$ unter 40 ng/kg/min nicht signifikant verschieden von den Meßergebnissen nach Salbutamol. Nur für den $MEF_{50}$-Wert nach 20 Minuten Infusion fand sich ein signifikanter Unterschied zwischen 40 und 60 ng/kg/min Urodilatin. 20 ng/kg/min Urodilatin zeigte einen Anstieg allein des $MEF_{75}$-Meßwertes mit Erreichen einer Irrtumswahrscheinlichkeit von $p < 0,05$ nach 10 und 30 Minuten Infusion. Ein signifikanter Effekt von Salbutamol war in allen Dosisgruppen nachweisbar.

In den Abb. 10 bis 12 wird der Verlauf der Kreislaufparameter dargestellt.

Das Abklingen einer Streßreaktion ausgelöst durch die körperliche Untersuchung wird durch die Reduktion der systolischen und diastolischen Blutdruckwerte von der Basis-Messung zur Bestimmung vor Infusionsbeginn dokumentiert. Während der Infusion kam es in keiner Dosisgruppe zu einer signifikanten Veränderung der Blutdruckwerte. Nur 20 und 30 Minuten nach Ende der Infusion von 40 ng/kg/min Urodilatin war ein gegenüber den Ausgangswerten (pre) signifikanter Abfall des systolischen Blutdrucks nachweisbar.

Die Herzfrequenz zeigte bereits für die Basis-Messung signifikante Unterschiede zwischen den Gruppen. Nach 40 Minuten Infusion war im Vergleich mit den jeweiligen Ausgangswerten ein signifikanter Anstieg in allen drei Dosisgruppen nachweisbar. Nur in der Gruppe mit 60 ng/kg/min Urodilatin hielt dieser signifikante Effekt bis 30 Minuten nach Ende der Infusion an. Nach Inhalation von 1,25 mg Salbutamol konnte in allen Gruppen ein signifikanter Anstieg der Herzfrequenz dokumentiert werden. Es wurden Mittelwerte signifikant oberhalb der Urodilatin-Maxima erreicht.

Die Nebenwirkungen von Urodilatin zeigten keine Korrelation zu der Höhe der infundierten Dosis. Ein Patient klagte über Kopfschmerzen unter 20 ng/kg/min, bei 3 von 18 Asthmatikern (40 ng/kg/min: 2 Patienten, 60 ng/kg/min: 1 Patient) kam es zum passageren Auftreten einer Bradykardie. Eine medikamentöse Intervention war bei keinem Patienten notwendig.

Ein diuretischer Effekt konnte bei 7 Patienten (20 ng/kg/min: 2, 40 ng/kg/min: 3, 60 ng/kg/min: beobachtet werden, während 8 von 18 Patienten (20 ng/kg/min: 3, 40 ng/kg/min: 2, 60 ng/kg/min:3) überhaupt keine Nebenwirkungen angaben.

3. Diskussion

Die intravenöse Infusion von 40 und 60 ng/kg/min Urodilatin über 40 Minuten führte zu einer signifikanten Bronchodilatation bei Patienten mit einem klinisch stabilen Asthma bronchiale. Der Anstieg von PEF, $MEF_{75}$, $MEF_{50}$ und $MEF_{25}$ reflektiert im Vergleich mit den Werten nach Salbutamol-Inhalation eine zentral betonte, aber auch periphere Wirkung. Dabei ist die Möglichkeit zu berücksichtigen, daß die Lokalisation des bronchodilatatorischen Effekts durch die unterschiedlichen Applikationsarten (intravenös/inhalativ) beeinflußt wurde.

60 ng/kg/min Urodilatin zeigte deutlicher noch als 40 ng/kg/min eine maximale Verbesserung der Lungenfunktionswerte ohne signifikante Unterschiede zu den Ergebnissen nach Salbutamol-Inhalation. Andererseits war mit Ausnahme des $MEF_{50}$ 20 Minuten nach Beginn der Infusion keine signifikante Differenz zwischen 60 und 40 ng/kg/min Urodilatin nachweisbar. Signifikante Veränderungen der systolischen und diastolischen Blutdruckwerte während der Infusion der drei Urodilatin-Dosen konnten nicht objektiviert werden. Die Herzfrequenz zeigte nach 40 Minuten Infusion einen signifikanten Anstieg in allen drei Gruppen. Nur unter 60 ng/kg/min war diese Veränderung bis zu 30 Minuten nach Ende der Infusion konstant signifikant nachweisbar. Auch die Salbutamol-Inhalation führt zu einer Zunahme der Herzfrequenz mit signifikant höheren Mittelwerten im Vergleich zu den Maxima unter Urodilatin-Infusion

Der Herzfrequenzanstieg unter 60 ng/kg/min muß als eine Reflextachykardie bedingt durch die vasodilatatorische Wirkung von Urodilatin interpretiert werden. Um das bronchodilatatorische Potential von Urodilatin ohne gleichzeitige indirekte Effekte durch eine Sympathikotonuserhöhung beurteilen zu können, sollte eine Dosierung von 40 ng/kg/min entsprechend 11,4 pmol/kg/min gewählt werden. Zum direkten Vergleich mit ANP und den Angaben in der Literatur wäre eine Dosis von 10 pmol/kg/min Urodilatin empfehlenswert. Eine Verkürzung der Infusionsdauer auf 30 Minuten scheint ohne signifikante Auswirkungen auf die erreichbaren Urodilatin-Maxima möglich zu sein.

Bei 7 von 18 Probanden wurde eine Diurese durch Urodilatin induziert. Für Patienten mit einem unkomplizierten Asthma bronchiale ohne Hinweise auf eine Rechtsherzinsuffizienz stellt dieser Effekt eine Nebenwirkung dar. Wird Urodilatin allerdings zur Behandlung einer chronisch obstruktiven Lungenerkrankung (COLD) eingesetzt, bei der sich im Verlauf eine Schwäche des rechten Herzens als Folge einer pulmonalen Hypertonie entwickelt hat, kann die Diurese als erwünschte zusätzliche therapeutische Wirkung genutzt werden.

**Patentansprüche**

1. Verwendung einer pharmazeutischen Zusammensetzung, die Urodilatin als Wirkstoff und gegebenenfalls pharmazeutisch übliche Verdünnungs-, Träger, Füll- oder Hilfsstoffe enthält, zur Herstellung eines Arzneimittels zur Behandlung von Lungen- oder/und Bronchialerkrankungen.

2. Verwendung nach Anspruch 1 zur Herstellung eines Arzneimittels zur Behandlung von obstruktiven Atemwegserkrankungen.

3. Verwendung nach Anspruch 1 oder 2, dadurch gekennzeichnet, daß man ein parenteral intravenös oder inhalatorisch verabreichbares Arzneimittel herstellt.

**4.** Verwendung nach einem der Ansprüche 1 bis 3, dadurch gekennzeichnet, daß eine Dosierungseinheit des Arzneimittels 5 ng bis 1000 µg Urodilatin/kg Körpergewicht entspricht.

**5.** Verwendung nach Ansprpch 4, dadurch gekennzeichnet, daß eine Dosierungseinheit des Arzneimittels 10 ng bis 100 µg Urodilatin/kg Körpergewicht entspricht.

## Claims

**1.** Use of a pharmaceutical composition containing urodilatin as the active ingredient and optionally pharmaceutically usual diluents, vehicles, fillers or auxiliary agents for the preparation of a medicament for the treatment of lung and/or bronchial diseases.

**2.** The use according to claim 1 for the preparation of a medicament for the treatment of obstructive respiratory tract diseases.

**3.** The use according to claim 1 or 2, characterized in that a parenterally, intravenously or inhalatorily, administer-able medicament is prepared.

**4.** The use according to any of claims 1 to 3, characterized in that a unit dose of the medicament corresponds to from 5 ng to 1000 µg of urodilatin/kg of body weight.

**5.** The use according to claim 4, characterized in that a unit dose of the medicament corresponds to from 10 ng to 100 µg of urodilatin/kg of body weight.

## Revendications

**1.** Utilisation d'une composition pharmaceutique contenant de l'urodilatine comme principe actif et, éventuellement, des diluants, des véhicules, des charges ou des adjuvants pharmaceutiquement usuels, pour la préparation d'un médicament pour le traitement de pneumopathies et/ou de bronchopathies.

**2.** Utilisation selon la revendication 1 pour la préparation d'un médicament pour le traitement de troubles respiratoires obstructifs.

**3.** Utilisation selon la revendication 1 ou 2, caractérisée en ce qu'on prépare un médicament pouvant être administré par voie parentérale par injection intraveineuse ou par inhalation.

**4.** Utilisation selon l'une des revendications 1 à 3, caractérisée en ce qu'une dose unitaire du médicament correspond à 5 ng à 1 000 µg d'urodilatine/kg de poids corporel.

**5.** Utilisation selon la revendication 4, caractérisée en ce qu'une dose unitaire du médicament correspond à 10 ng à 100 µg d'urodilatine/kg de poids corporel.

Abb.1

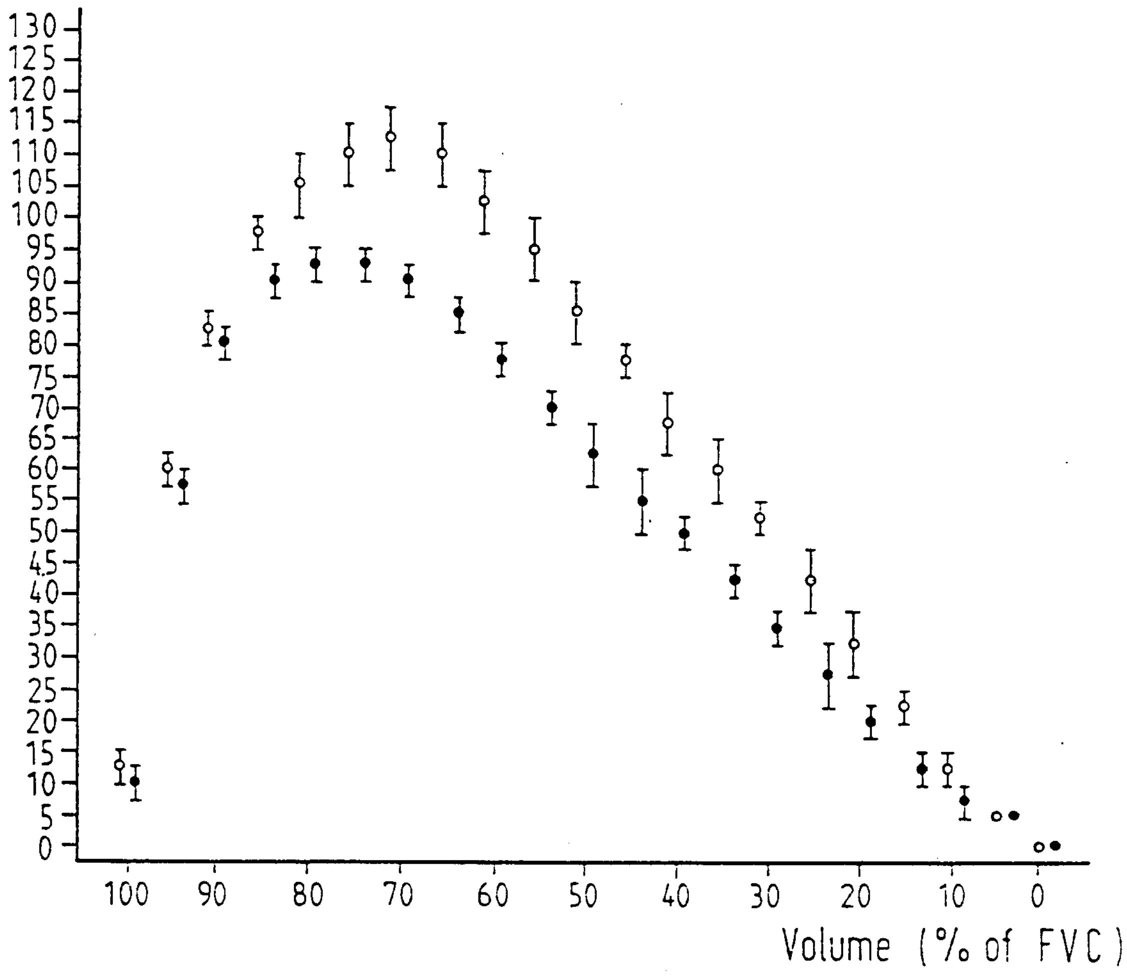
130
125
120
115
110
105
100
95
90
85
80
75
70
65
60
55
50
45
40
35
30
25
20
15
10
5
0
100
90
80
70
60
50
40
30
20
10
0
Volume (% of FVC)

Abb. 2

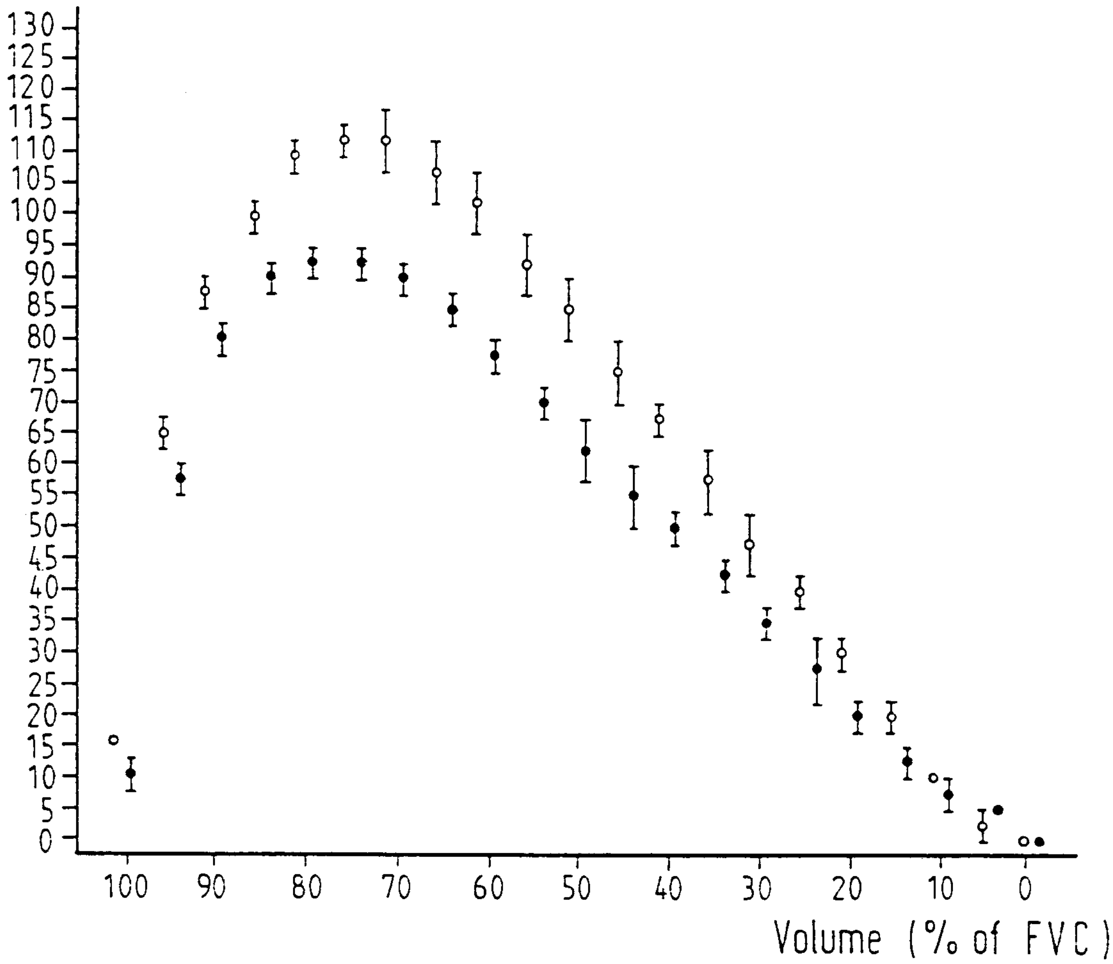
130
125
120
115
110
105
100
95
90
85
80
75
70
65
60
55
50
45
40
35
30
25
20
15
10
5
0
100 90 80 70 60 50 40 30 20 10 0
Volume (% of FVC)

Abb. 3

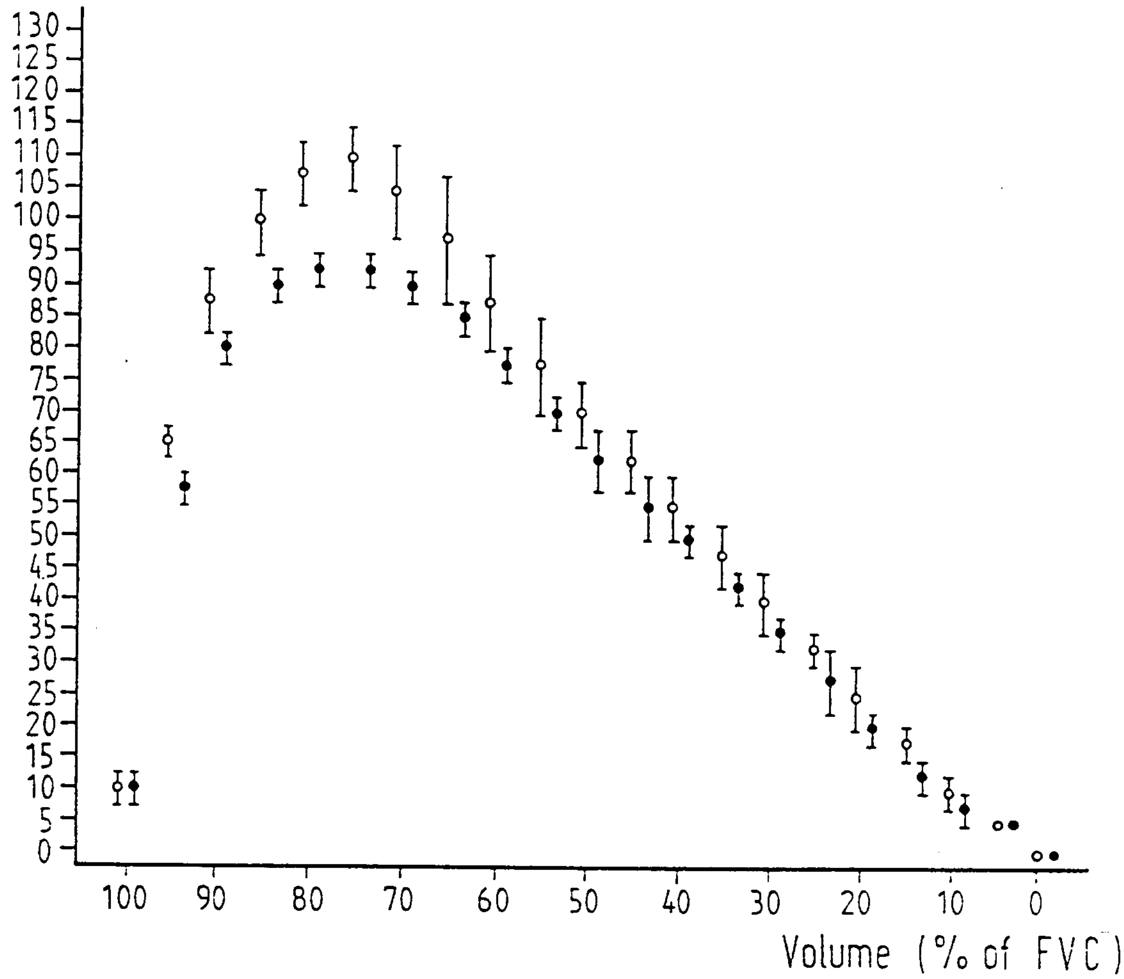
130
125
120
115
110
105
100
95
90
85
80
75
70
65
60
55
50
45
40
35
30
25
20
15
10
5
0
100
90
80
70
60
50
40
30
20
10
0
Volume (% of FVC)

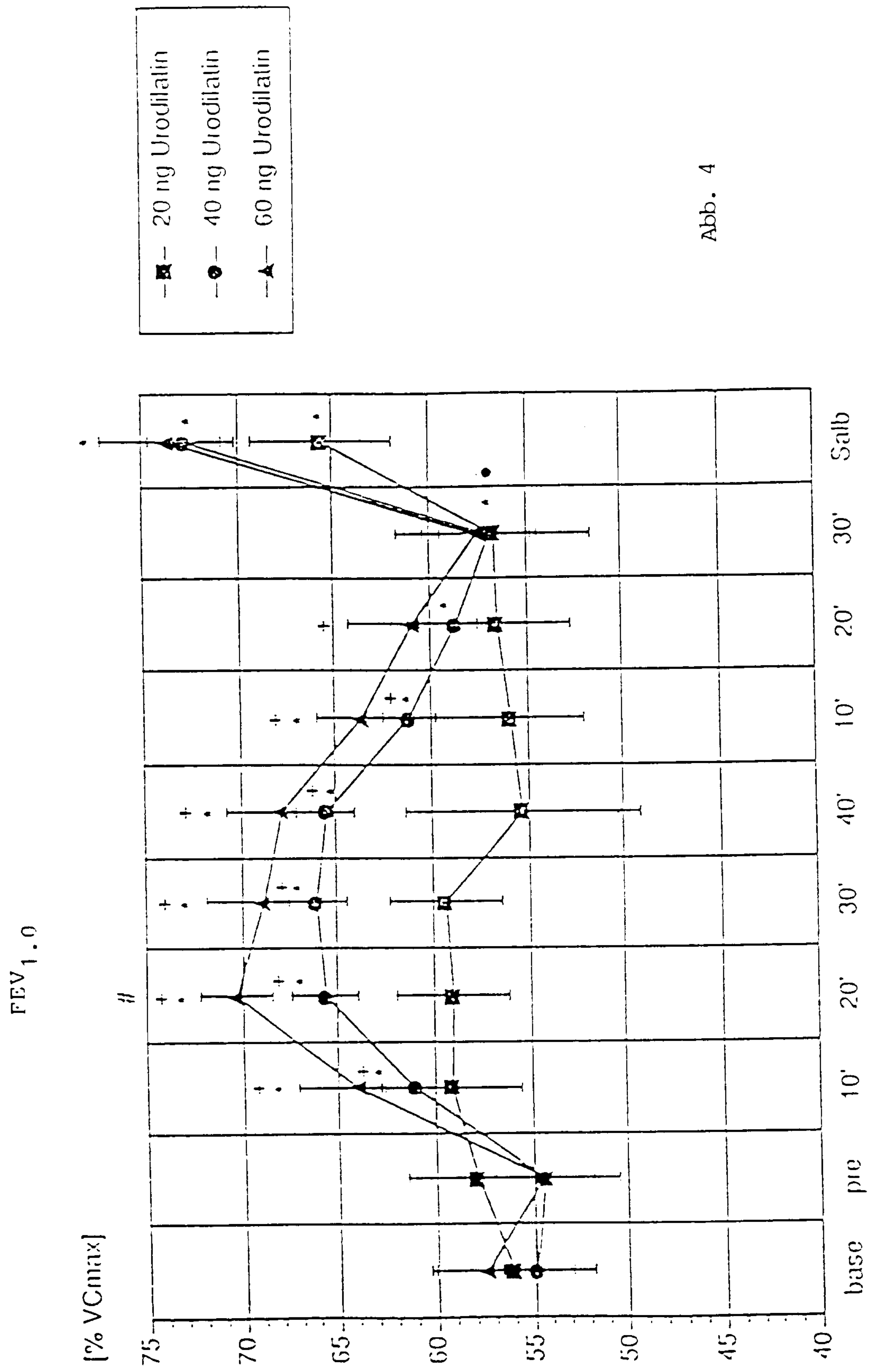
FEV1.0
[% VCmax]
75
70
65
60
55
50
45
40
base
pre
10'
20'
30'
40'
10'
20'
30'
Salb
#
20 ng Urodilatin
40 ng Urodilatin
60 ng Urodilatin

Abb. 4

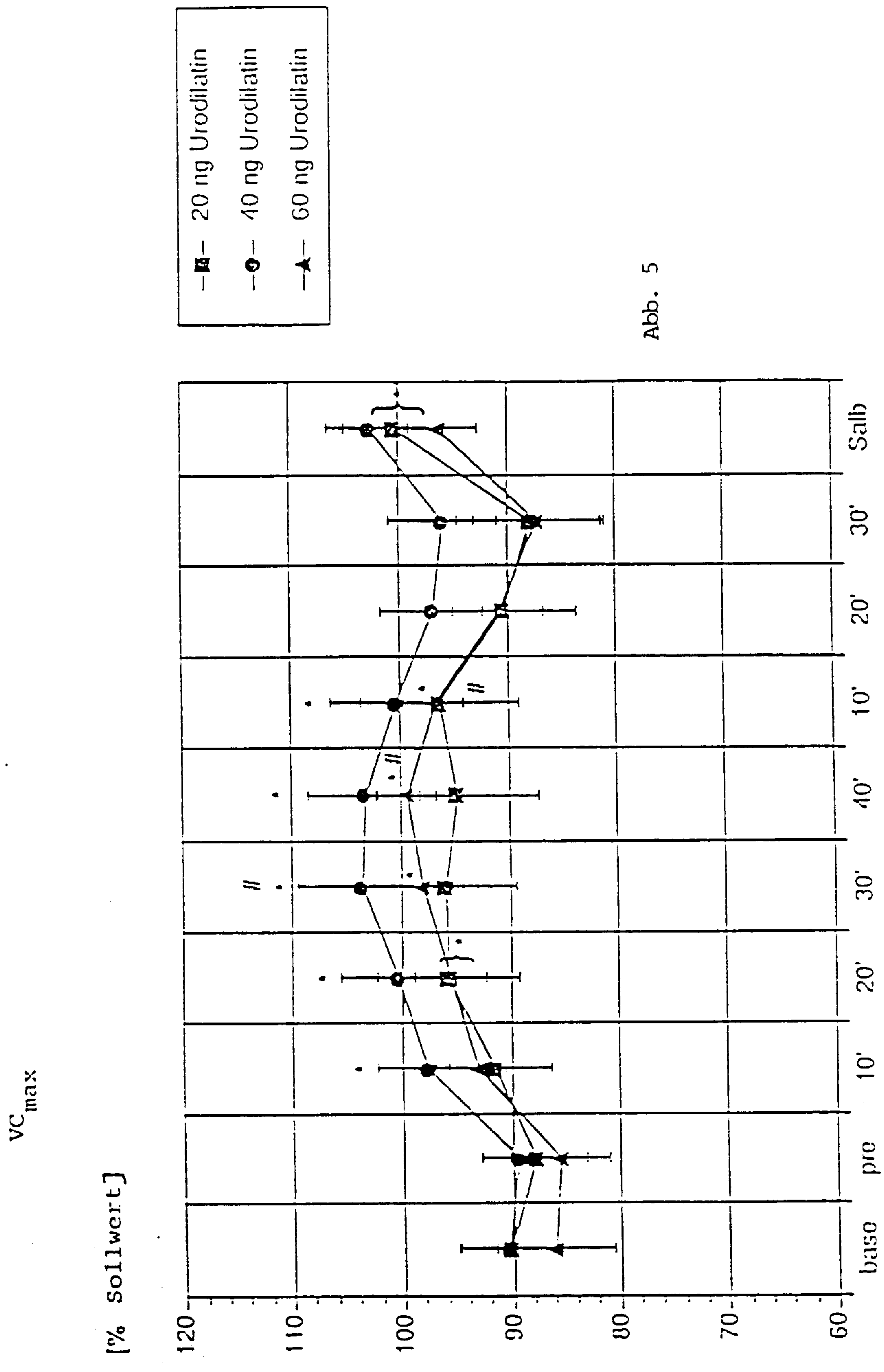
VCmax
[% Sollwert]
120
110
100
90
80
70
60
base
pre
10'
20'
30'
40'
10'
20'
30'
Salb
20 ng Urodilatin
40 ng Urodilatin
60 ng Urodilatin

Abb. 5

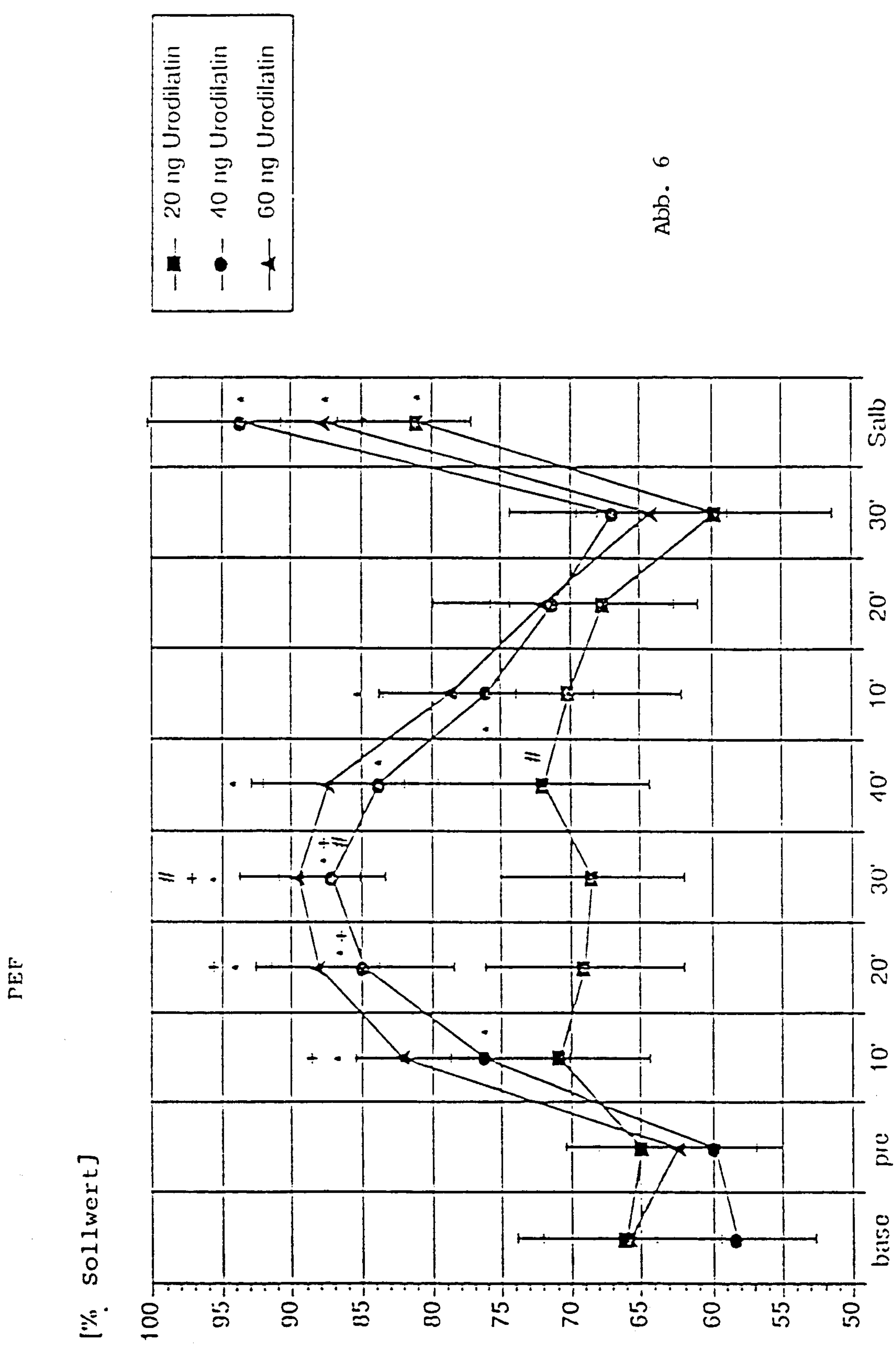
PEF
[% Sollwert]
100
95
90
85
80
75
70
65
60
55
50
base
pre
10'
20'
30'
40'
10'
20'
30'
Salb
20 ng Urodilatin
40 ng Urodilatin
60 ng Urodilatin

Abb. 6

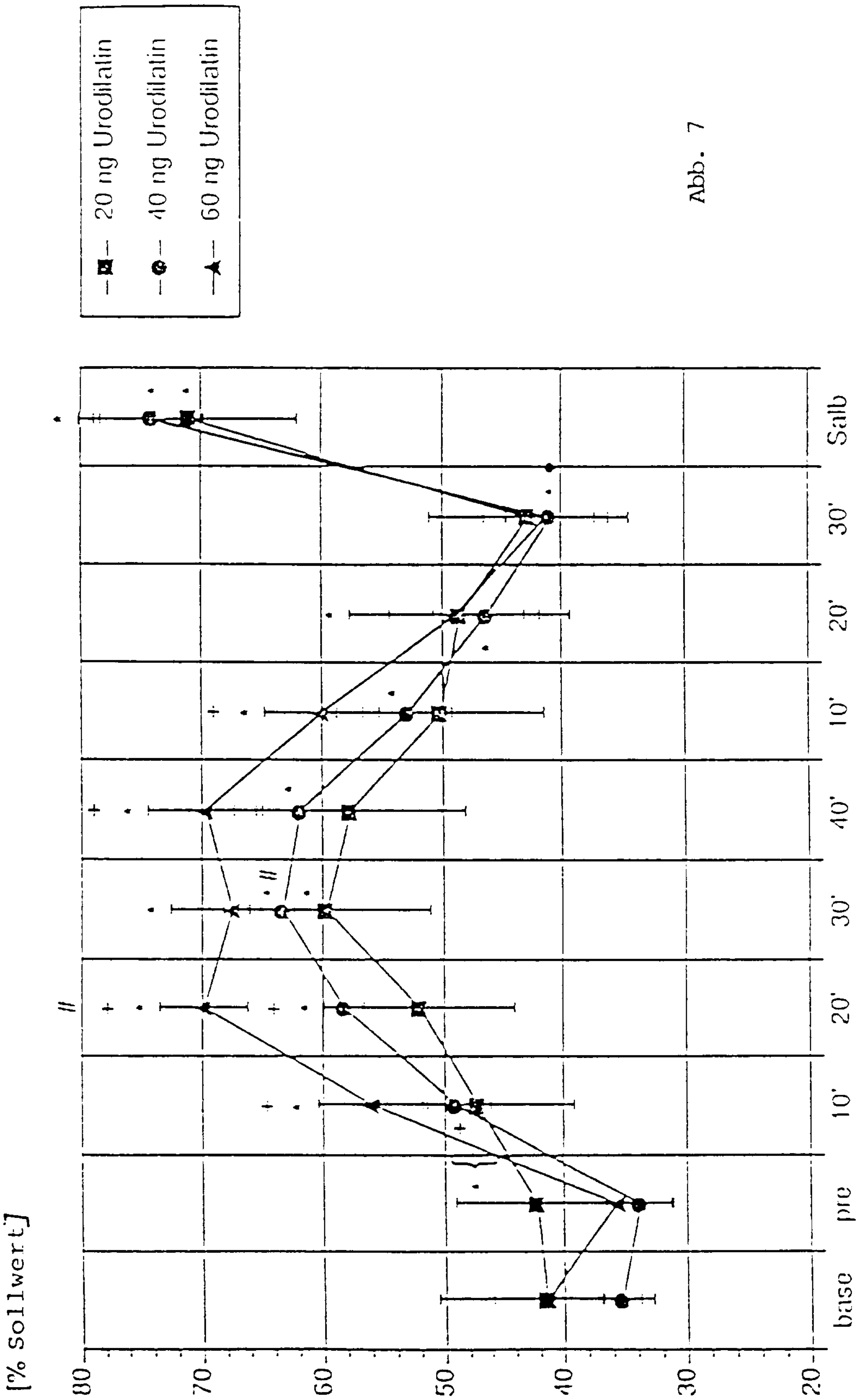
[% Sollwert]
MEF75
80
70
60
50
40
30
20
base
pre
10'
20'
30'
40'
10'
20'
30'
Salb
20 ng Urodilatin
40 ng Urodilatin
60 ng Urodilatin

Abb. 7

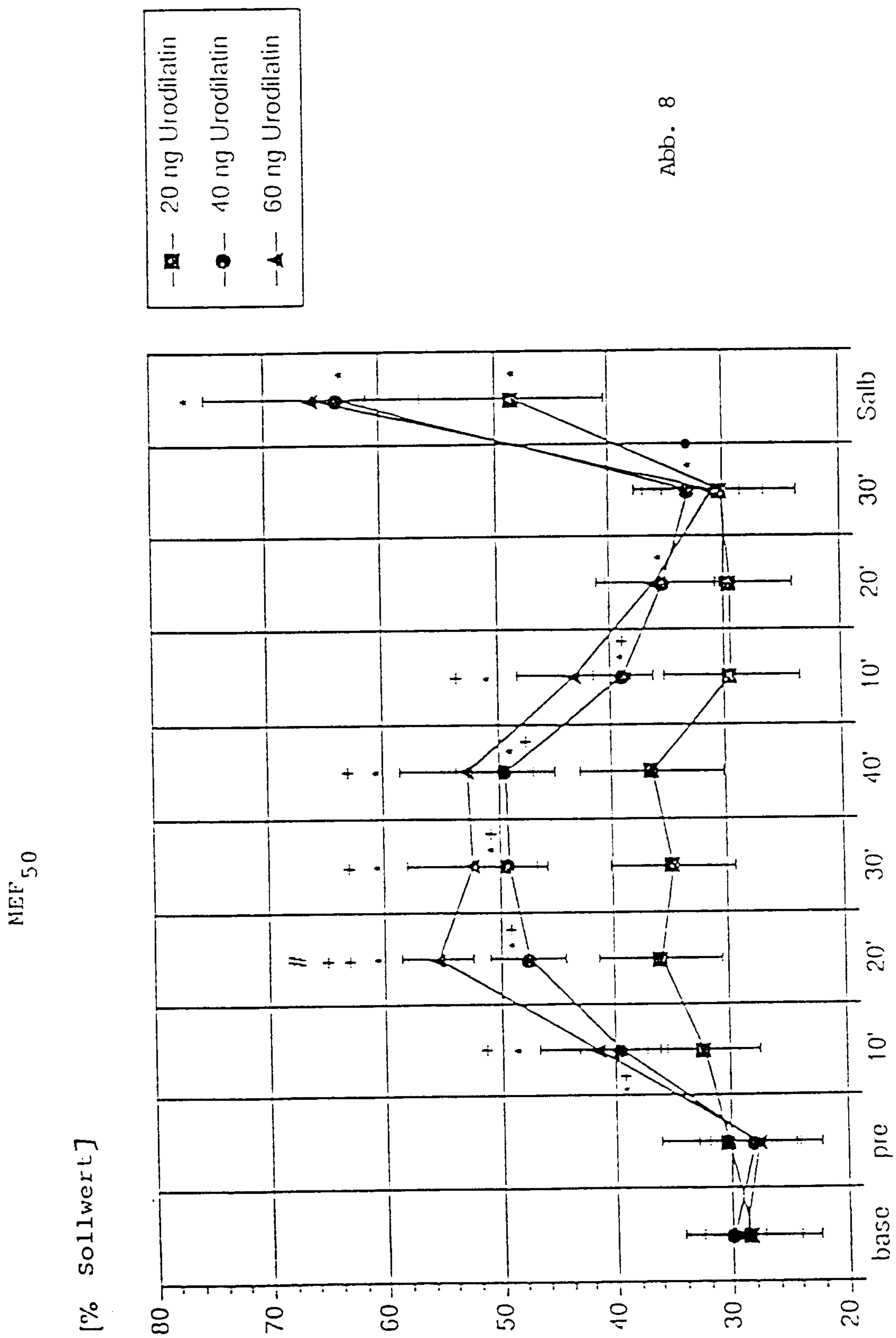
MEF50
[% Sollwert]
80
70
60
50
40
30
20
base
pre
10'
20'
30'
40'
10'
20'
30'
Salb
20 ng Urodilatin
40 ng Urodilatin
60 ng Urodilatin

Abb. 8

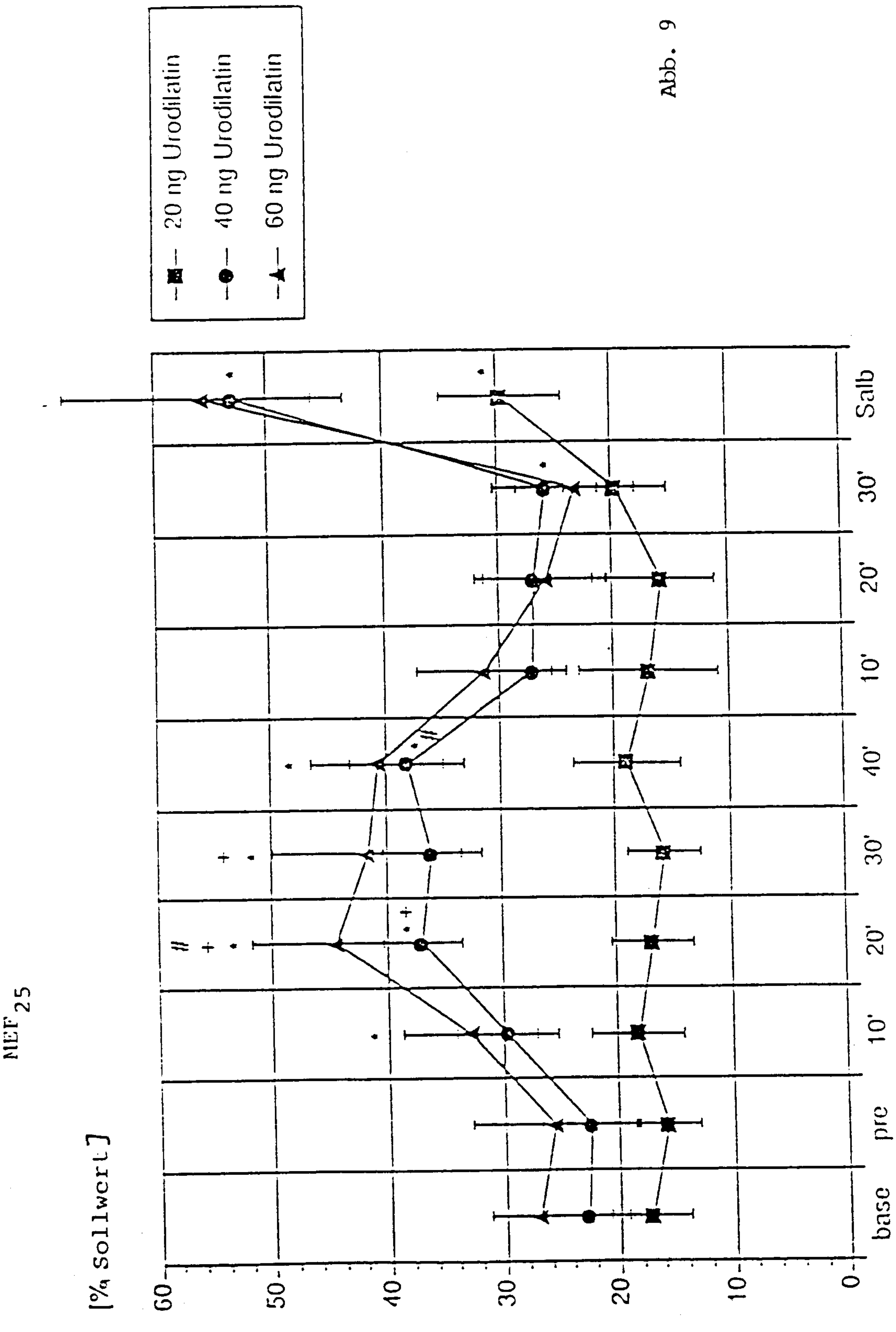
MEF25
[% Sollwert]
60
50
40
30
20
10
0
base
pre
10'
20'
30'
40'
10'
20'
30'
Salb
20 ng Urodilatin
40 ng Urodilatin
60 ng Urodilatin

Abb. 9

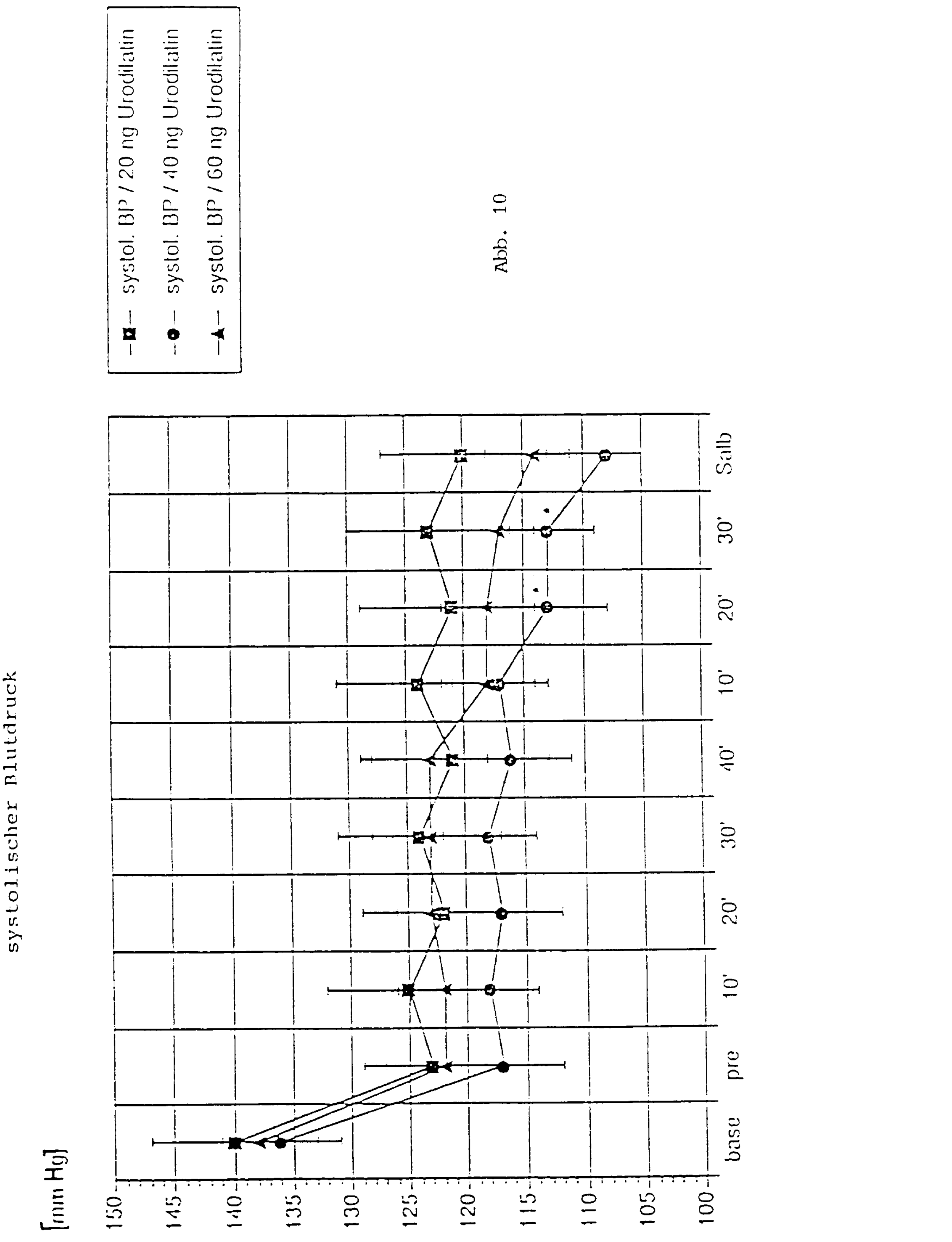
systolischer Blutdruck
[mm Hg]
150
145
140
135
130
125
120
115
110
105
100
base
pre
10'
20'
30'
40'
10'
20'
30'
Salb
systol. BP / 20 ng Urodilatin
systol. BP / 40 ng Urodilatin
systol. BP / 60 ng Urodilatin

Abb. 10

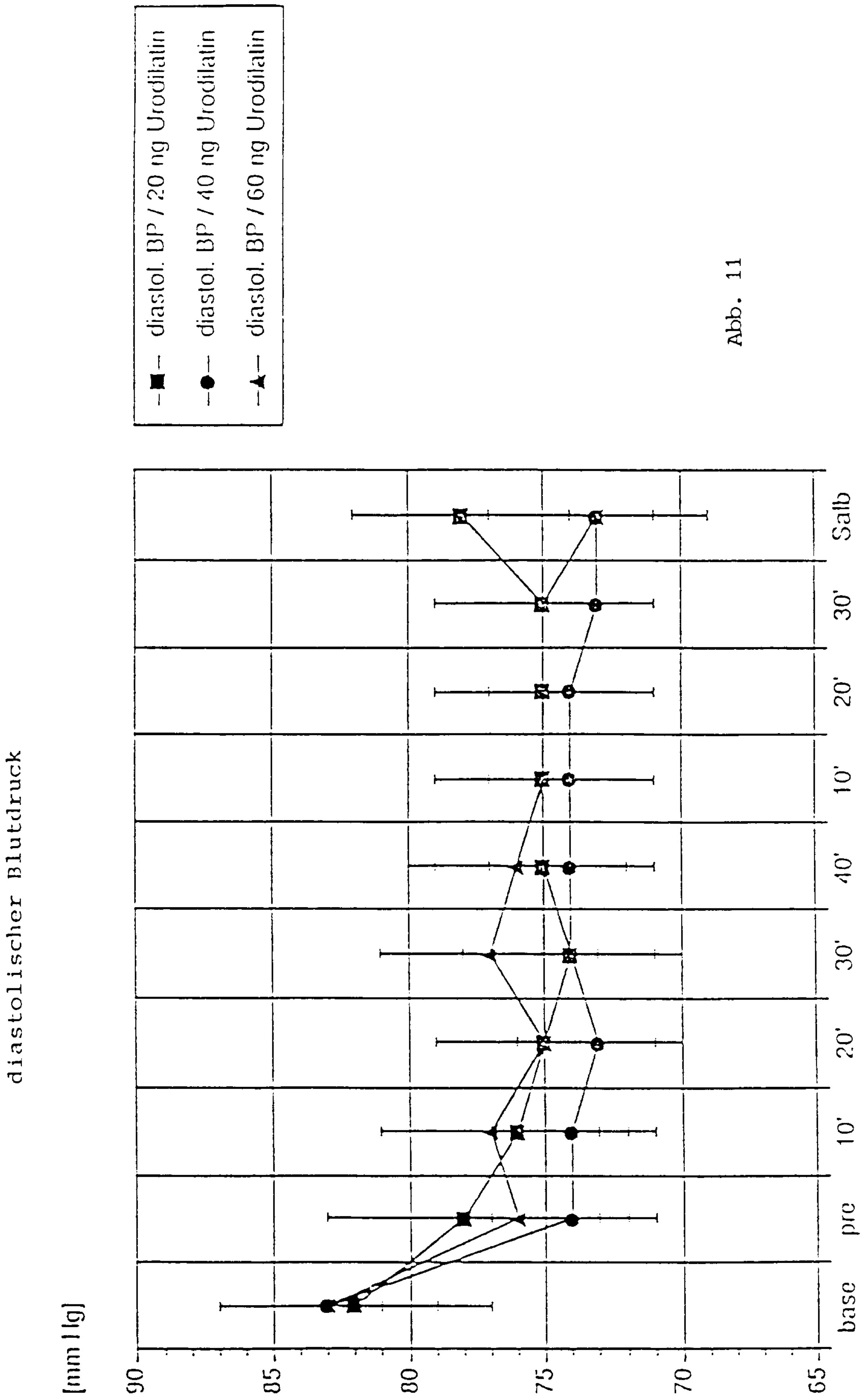
diastolischer Blutdruck
[mm Hg]
90
85
80
75
70
65
base
pre
10'
20'
30'
40'
10'
20'
30'
Salb
diastol. BP / 20 ng Urodilatin
diastol. BP / 40 ng Urodilatin
diastol. BP / 60 ng Urodilatin

Abb. 11

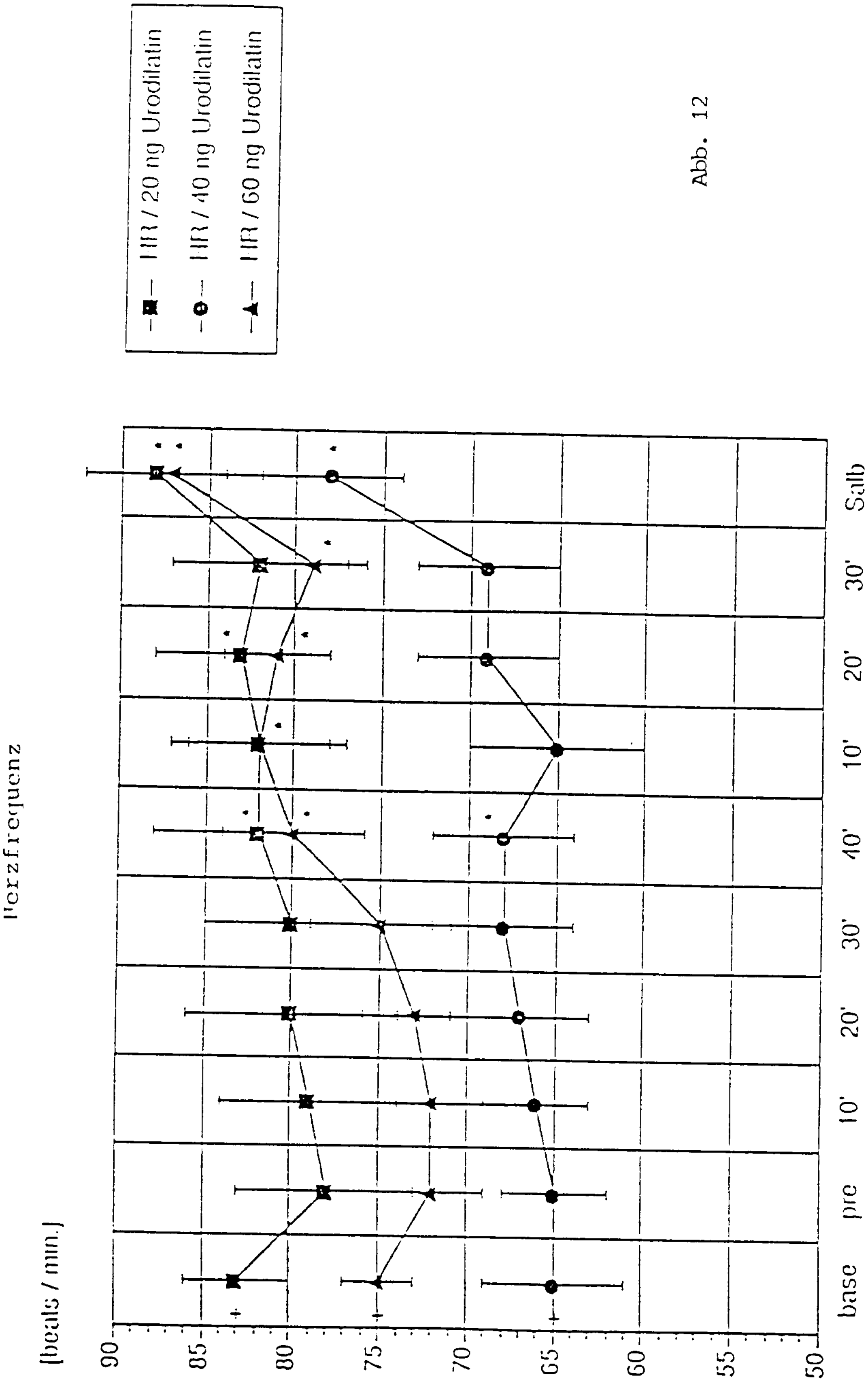
Herzfrequenz
[beats / min.]
90
85
80
75
70
65
60
55
50
base
pre
10'
20'
30'
40'
10'
20'
30'
Salb
HR / 20 ng Urodilatin
HR / 40 ng Urodilatin
HR / 60 ng Urodilatin

Abb. 12